# EUROPEAN PATENT APPLICATION

(11) **EP 1 813 290 A1**
(43) Date of publication of application: **01.08.2007**
(21) Application number: 07007686.4
(22) Date of filing: 28.01.2005
(51) Int. Cl.: A61L 15/38, A61L 15/60

(54) **Skin dressings comprising hydrated hydrogel lactate ions and glucose**

(30) Priority: 30.01.2004 EP 04250508; 15.12.2004 GB 0427444
(62) Divisional of application: 05702061.2
(71) Applicant: Insense Limited, Sharnbrook, Bedfordshire MK44 1LQ (GB)
(72) Inventor: Davis, Paul James, Felmersham Bedford, MK43 7EX (GB); Austin, Andrew John, Irchester Northants, NN9 7DJ (GB); Jezek, Jan, Stanwick Horthants, NN9 6TE (GB)
(74) Representative: Willett, Christopher David

(57) **Abstract**

A skin dressing comprises a hydrated hydrogel material in combination with a source of oxygen or an oxidising agent such as hydrogen peroxide, optionally including a source of zinc ions and a source of iodide ions. The dressing has beneficial effects on skin.

## Description

### Field of the Invention

This invention relates to skin dressings for application to a part of a human or animal body for treatment of skin (for therapeutic or cosmetic purposes), and relates particularly (but not exclusively) to wound dressings for treatment of compromised skin, particularly skin lesions, i. e. any interruption in the surface of the skin, whether caused by injury or disease, including skin ulcers, burns, cuts, punctures, lacerations, blunt traumas, acne lesions, boils etc.

### Background to the Invention

Skin and wound dressings are designed to undertake a number of important functions to aid the process of healing. Experts agree on most of the functions that an ideal dressing should provide, and these include:
- Donation of moisture to dry wounds
- Absorption of excess fluid from weeping wounds
- Maintenance of a moist environment around the wound bed
- Binding of water sufficiently well to prevent maceration (water-logging) of the normal tissue
- Aiding debridement (removal of dead tissue and scar material)
- Prevention of infection and provision of a barrier to escaping or invading microbes
- Killing infecting microbes
- Cushioning against further physical trauma
- Maintaining an optimum temperature through thermal insulation
- Allowing ingress of plentiful oxygen
- Soothing painful and inflamed open wound sites
- Flexibly adapting to the shape of the wound site
- Keeping its physical integrity so that fragmented dressing debris is not left in the wound
- Exerting no cytotoxic nor physically damaging effects on the healing cells.

In addition, the handling and physical design characteristics should make the dressing easy to use and comfortable to wear. For storage and distribution purposes, the dressing should be stable at ambient temperatures, and robust. Ideally it should be simple to manufacture, in order to allow its production and sale at a price that is affordable for widespread use.

These and other demands make the design of an ideal wound dressing almost impossible. To date, all wound dressings are a compromise, such that none offers all of the much desired characteristics in one product. For this reason, there are numerous different wound dressings on the market, and the typical nurse caring for patients with wounds needing professional care will select different dressings for different wounds and for wounds at different phases of the wound healing process. Manufacturers are constantly seeking new ways to make more effective wound dressings, which means that they are trying to make dressings that incorporate more of the characteristics and functions listed above. With the achievement of each new benefit, the cause of improved patient welfare is advanced, as the result of faster healing, reduction of pain and improvement in the quality of life. Medical care in general can benefit from such progress. Although these advanced, "active" dressings usually cost more, they can reduce the overall time during which a wound needs attention and reduce the amount of nursing time devoted to frequent changes of dressing. This drives down the huge cost borne by modern society in caring for wounds.

Our British Patent Application No. 0313217.2 filed on 9th June 2003 and International Application No. PCT/GB2004/002374 filed 4th June 2004 disclose a skin dressing comprising a first dressing component carrying oxidoreductase enzyme in dried condition; and a second dressing component carrying a source of water, such that when the first and second dressing components are placed in fluid communication with each other, water migrates from the second component towards the first component and acts to hydrate enzyme carried by the first component, at least at the surface of the first component.

In one embodiment, the second dressing component comprises a hydrogel formulated to include the following reagents by weight:
20% sodium AMPS (2-acrylamido-2-methylpropanesulfonic acid, sodium salt (Lubrizol, code 2405))
0.2% poly ethylene glycol 400 diacrylate (UCB Chemicals)
0.01% photoinitiator (1-hydroxycyclohexyl phenyl ketone (Aldrich))
20% glucose (Fisher)
0.1% zinc lactate (Sigma)
0.05% potassium iodide (Fisher)
To 100% with DI-water.

It has now surprisingly been appreciated that such a hydrogel may to advantage be used on its own for skin treatment, not necessarily in combination with a first dressing component as disclosed in our earlier applications.

### Summary of the Invention

In one aspect the present invention provides a skin dressing comprising a hydrated hydrogel material comprising a source of lactate ions and a supply of glucose.

In a further aspect, the invention provides a skin dressing comprising a hydrated hydrogel material comprising a source of lactate ions and a supply of glucose, excluding a hydrated hydrogel comprising the following reagents by weight: 20% sodium AMPS (2-acrylamido-2-methylpropanesulfonic acid, sodium salt (Lubrizol, code 2405)), 0.2% poly ethylene glycol 400 diacrylate (UCB Chemicals), 0.01% photoinitiator (1-hydroxycyclohexyl phenyl ketone (Aldrich)), 20% glucose (Fisher), 0.1% zinc lactate (Sigma), 0.05% potassium iodide (Fisher) and to 100% with DI-water.

In another aspect, the invention provides a skin dressing comprising a hydrated hydrogel material comprising a source of lactate ions and a supply of glucose, wherein the glucose is present in an amount of less than 20% by weight of the weight of the hydrated hydrogel material.

A hydrated hydrogel means one or more water-based or aqueous gels, in hydrated form.

A hydrated hydrogel can act to absorb water and other materials exuded from a wound site, enabling the dressing to perform a valuable and useful function by removing such materials from a wound site. The hydrated hydrogel also provides a source of moisture, that can act in use to maintain a wound site moist, aiding healing.

The hydrated hydrogel conveniently comprises hydrophilic polymer material. Suitable hydrophilic polymer materials include polyacrylates and methacrylates, e.g. as supplied by First Water Ltd in the form of proprietary hydrogels, including poly 2-acrylamido-2-methylpropane sulphonic acid (polyAMPS) or salts thereof (e.g. as described in WO 01/96422), polysaccharides e.g. polysaccharide gums particularly xanthan gum (e.g. available under the Trade Mark Keltrol), various sugars, polycarboxylic acids (e.g. available under the Trade Mark Gantrez AN-169 BF from ISP Europe), poly(methyl vinyl ether co-maleic anhydride) (e.g. available under the Trade Mark Gantrez AN 139, having a molecular weight in the range 20,000 to 40,000), polyvinyl pyrrolidone (e.g. in the form of commercially available grades known as PVP K-30 and PVP K-90), polyethylene oxide (e.g. available under the Trade Mark Polyox WSR-301), polyvinyl alcohol (e.g. available under the Trade Mark Elvanol), cross-linked polyacrylic polymer (e.g. available under the Trade Mark Carbopol EZ-1), celluloses and modified celluloses including hydroxypropyl cellulose (e.g. available under the Trade Mark Klucel EEF), sodium carboxymethyl cellulose (e.g. available under the Trade Mark Cellulose Gum 7LF) and hydroxyethyl cellulose (e.g. available under the Trade Mark Natrosol 250 LR).

Mixtures of hydrophilic polymer materials may be used in a gel.

In a hydrated hydrogel of hydrophilic polymer material, the hydrophilic polymer material is desirably present at a concentration of at least 1%, preferably at least 2%, more preferably at least 5%, yet more preferably at least 10%, or at least 20%, desirably at least 25% and even more desirably at least 30% by weight based on the total weight of the gel. Even higher amounts, up to about 40% by weight based on the total weight of the gel, may be used.

Good results have been obtained with use of a hydrated hydrogel of poly AMPS or salts thereof in an amount of about 30% by weight of the total weight of the gel.

By using a gel comprising a relatively high concentration (at least 2% by weight) of hydrophilic polymer material, the gel can function particularly effectively to take up water in use of the dressing, e.g. from serum exudates while in contact with a wound. Because the gel is an aqueous system, use of the dressing does not have the effect of inducing an overall dryness of the wound which would be undesirable. This is because water vapour pressure is maintained in the enclosed environment surrounding the skin in use of the dressing. The gel thus functions as an absorbent entity for the removal of moisture, e.g. wound exudate, that also provides a helpful background level of excess moisture.

The water-uptake capacity of a hydrated hydrogel, including a high concentration gel, enables the dressing to aid wound healing by removing substantial amounts of exudates, swelling-up as it does so. By using a carefully formulated, ready-hydrated gel, the wound is prevented from reaching a state of unhelpful dryness. Ready hydration also ensures the quick formation of an aqueous liquid interface between the dressing and the wound, thus preventing adhesion, which otherwise would interfere with easy lifting of the dressing when it has to be replaced. A good aqueous liquid interface between the wound and the dressing is also important in allowing any beneficial products carried in the gel to enter the wound through all of the available surface.
The hydrated hydrogel material is typically in the form of a solid layer, sheet or film of material that is typically cross-linked, and that may incorporate a mechanical reinforcing structure. The size and shape of the layer, sheet or film can be selected to suit the intended use of the dressing. Thicknesses in the range 0.01 to 1.0 mm, preferably 0.05 to 0.5 mm are particularly suitable.

Alternatively, the hydrated hydrogel may be in the form of an amorphous gel not having a fixed form or shape, that can be deformed and shaped in three dimensions, including being squeezed through a nozzle. Amorphous gels are typically not cross-linked or have low levels of cross-linking: A shear-thinning amorphous gel may be used. Such a gel is liquid when subjected to shear stress (e.g. when being poured or squeezed through a nozzle) but set when static. Thus the gel may be in the form of a pourable or squeezable component that may be dispensed, e.g. from a compressible tube or a syringe-like dispenser, comprising a piston and cylinder, typically with a nozzle of about 3 mm diameter. Such a gel may be applied in the form of a surface layer, or into a wound cavity as a fully conformable gel that fills the available space and contacts the wound surface.

A typical example of an amorphous gel formulation is: 15% w/w AMPS (sodium salt), 5% w/w glucose, 0.05% w/w potassium iodide, 0.1% zinc lactate, 0.19% polyethylene glycol diacrylate and 0.01 % hydroxycyclohexyl phenyl ketone, with the volume made up to 100% with analytical grade DI water. The reagents are thoroughly mixed and dissolved, then polymerised for between 30-60 seconds, using a UV-A lamp delivering approximately 100 mW/cm², to form the required hydrogel. This may be contained in plastic syringes from which the amorphous gel may then be dispensed from a syringe to a target site, as a surface layer or to fill a cavity.

The source of lactate ions may be any compound capable of releasing lactate ions or lactate-containing ions in water. The lactate ion (derived from lactic acid) is optically active and so may exist in two enantiomeric forms, L- and D-, and as a mixture of both enantiomers, known as a racemate. Any enantiomeric form, or any mixture of enantiomeric forms, is suitable for use herein. Convenient sources of lactate ions include sodium L-lactate, sodium D-lactate, sodium D, L-lactate and zinc L-lactate, although it is believed that any soluble lactate can be used as a source of lactate ions.

The lactate ions function as a pH buffering substance and as an anti-oxidant. Lactate ions are also believed to have an important role in creating an environment that stimulates or supports angiogenesis, the growth and regeneration of new blood vessels, as well as optimising the redox environment for cellular interaction. There may also be other beneficial effects of lactate in the wound environment, but these are not yet fully understood.

The lactate ions are suitably present in an amount of about 0.1% w/v.

The dressing desirably also includes a source of zinc ions. The source of zinc ions may be any compound capable of releasing zinc ions or zinc-containing ions in water. Suitable sources of zinc ions include, for example, zinc lactate, zinc chloride, zinc fluoride, and zinc sulphate.

The function of the zinc ions is as an anti-oxidant and as a general healing and skin benefit agent, with well-known soothing and anti-inflammatory effects. Zinc is an essential nutritional trace element which has numerous functions in the growth and repair of healthy tissues. In addition, zinc ions are known to form stabilising complexes with hydrogen peroxide, thus aiding delivery of hydrogen peroxide to the target site in embodiments discussed below involving hydrogen peroxide.

The zinc ions are suitably present in an amount of about 0.1% w/v.

A currently preferred source of zinc ions and lactate ions is zinc lactate, particularly zinc L-lactate.

The glucose functions to support the biosynthesis of glucose-containing tissue matrix polymers, such as hyaluronic acid, and as an energy source for metabolically active cells, with beneficial effects on wound healing.

The glucose is suitably present in an amount of at least 2.5%, preferably at least 5% by weight of the weight of the hydrated hydrogel material, with higher amounts also being possible. Good results have been obtained with a dressing including 5% by weight of glucose.

The dressing optionally includes a source of iodide ions, e.g. potassium iodide or sodium iodide. Iodide ions can be oxidised to elemental iodine in the presence of a suitable oxidising agent. Iodine is a known powerful antimicrobial agent with beneficial effects on skin, e.g. as disclosed in WO 01/28600 and WO 03/090800.

The iodide ions are suitably present in an amount in the range 0.05% to 0.2% w/v.

The skin dressing of the invention may be used on its own, being located on the skin of a human or animal, e.g. over a wound or on a region of skin to be treated for cosmetic or therapeutic purposes, e.g. for treatment of acne or other skin conditions. The lactate ions, glucose and optional zinc ions are observed to have beneficial effects on skin and wound healing.

Alternatively, the skin dressing or the invention may be used in combination with a source of oxygen or an oxidising agent, such as hydrogen peroxide. For example, the dressing may be used in combination with superposed material that generates hydrogen peroxide, such as layer comprising oxidoreductase enzyme. The oxidoreductase enzyme may be in dry condition, e.g. as disclosed in PCT/GB2004/002374, but is preferably in hydrated condition, e.g. as disclosed in WO 03/090800 and European Patent Application No. 04250508.1 filed 30th January 2004, preferably being included in a hydrated hydrogel, e.g. of materials as discussed above. The oxidoreductase enzyme catalyses reaction of an appropriate substrate with oxygen to produce hydrogen peroxide. Suitable oxidoreductase enzymes are listed in WO 03/090800. The currently preferred oxidoreductase enzyme is glucose oxidase, with the corresponding substrate being glucose. Thus, the glucose in the dressing of the invention acts as a substrate in this case for generation of hydrogen peroxide.

In another embodiment, the superposed layer may contain a supply of pre-formed hydrogen peroxide, or a hydrogen peroxide precursor substance.

Hydrogen peroxide is a known antimicrobial substance, with many beneficial properties. Where the dressing of the invention includes iodide ions, hydrogen peroxide reacts with iodide ions to generate molecular iodine, which also has beneficial effects on skin. In addition, the rapid decomposition of hydrogen peroxide in contact with tissues and tissue-fluids results in the release of oxygen which is available to help in the healing process and acts against anaerobic bacteria that may be present.

In a preferred aspect, the invention thus provides a skin dressing comprising a first hydrated hydrogel material including a source of lactate ions and a supply of glucose with optional sources of zinc ions and iodide ions; as discussed above, and a second hydrated hydrogel material comprising an oxidoreductase enzyme. The two hydrated hydrogel materials are preferably in the form of layers, sheets or films. The two hydrated hydrogels preferably comprise poly AMPS or salts thereof, desirably in an amount of about 30% by weight of the total weight of the hydrated hydrogels.

In this case the skin dressing is used by being located on the skin of a human or animal, as discussed above, with a superimposed hydrated hydrogel material comprising an oxidoreductase enzyme being located thereon. As well as the beneficial skin effects arising from the lower skin-contacting layer, beneficial effects also arise from generation of hydrogen peroxide and possibly also iodine.

The skin dressing of the invention may also be formulated or constructed in such a way as to control or regulate the rate of diffusion (and hence the effective dose) of hydrogen peroxide, eg by means of limited available water, by an abundance of hydrogen bonding groups in the gel structure, or by limiting the cross-sectional area of the wound-facing surface of the gel by the incorporation of a scrim that acts as a partial barrier.

The dressing conveniently includes, or is used with, a covering or outer layer for adhering the dressing to the skin of a human or animal subject in known manner.

The skin dressing (or components thereof) is desirably supplied in sterile, sealed, water-impervious packages, e.g. laminated aluminium foil packages.

### Example 1

The following composition is a skin treatment product of the form shown in Figure 6 of WO 03/090800, which comprises a glucose-containing hydrogel slab in accordance with the invention as a lower layer of the product, and an optional additional upper layer comprising a poly-AMPS hydrogel that incorporates glucose oxidase.

The hydrogel lower layer in accordance with the invention was formulated to include the following ingredients by weight:

| | |
|---|---|
| Water (ex Fisher, distilled, de-ionised, analytical grade) | 64.7% |
| Sodium AMPS (ex Lubrizol AMPS 2405 Monomer) | 30.0% |
| Polyethylene glycol diacrylate (PEG400 diacrylate, ex UCB Chemicals available as Ebecryl 11) | 0.19% |
| 1-hydroxycyclohexyl phenyl ketone (a photoinitiator, ex Aldrich) | 0.01% |
| Anhydrous glucose (enzyme substrate, ex Fisher) | 5.00% |
| Potassium iodide (ex Fisher) | 0.05% |
| Zinc L-lactate hydrate (ex Aldrich) | 0.10% |

The mixture was dispensed into casting trays containing either polyester scrim (polyester non-woven, open mesh support, available from HDK Industries Inc, Product Code 5722) or polyethylene net support, of dimensions 100mm x 100mm, to a depth of about 1.5mm. The polyethylene net support was fabricated from polyester staple fibres thermally bonded by a polyester resin - Product code 5722, from Castle Industries, Greenville, SC 9609, USA. The hydrogel was then set, by irradiation under a UV lamp, for up to 60 seconds and a power rating of approximately 100mW/cm². The hydrogel was then allowed to cool to 30°C or below.

The enzyme-containing hydrogel was formulated to include the following ingredients by weight:

| | |
|---|---|
| Water (ex Fisher, distilled, de-ionised, analytical grade) | 68.6% |
| Sodium AMPS (ex Lubrizol AMPS 2405 Monomer) | 15.0% |
| Ammonium AMPS (ex Lubrizol AMPS 2411 Monomer) | 15.0% |
| Polyethylene glycol diacrylate (PEG400 diacrylate, ex UCB Chemicals available as Ebecryl 11) | 0.19% |
| 1-hydroxycyclohexyl phenyl ketone (a photoinitiator, ex Aldrich) | 0.01% |
| Glucose oxidase (GOX, Biocatalysts, Pontypridd, Code G575P) | 0.035% |
| Zinc L-lactate hydrate (ex Aldrich) | 1.0% |
| Pluronic P65 (block co-polymer of ethylene oxide and propylene oxide, HO-[CH2CH2O]x-[CH2CHCH3O]y-[CH2CH2O]y-H, average MW 3400 (BASF) | 0.15% |

The mixture was dispensed into casting trays containing polyester scrim (polyester non-woven, open mesh support, available from HDK Industries Inc, Product Code 5722) of dimensions 100mm x 100mm, to a depth of about 1.0mm. The hydrogel was then set, by irradiation under a UV lamp, for up to 30 seconds (typically 25 seconds), and a power rating of approximately 100mW/cm². The hydrogel was then allowed to cool to 30°C or below.

The resulting gel layers were packaged separately in pouches or enclosures impermeable to water or water-vapour, e.g. made of laminated aluminium foil pouches as supplied by Sigma (code Z183407).

In use, eg on a wound, the wound contact layer can be used on its own to provide beneficial effects on a wound environment. Alternatively, the enzyme-containing hydrogel and the glucose-containing hydrogel can be brought together on a wound surface, one overlying the other.

An oxygen-permeable and moisture-permeable covering or overlay such as of polyurethane may be located over the enzyme-containing hydrogel and may be adhered to the skin by means of e.g. acrylic adhesive provided on the lower face of the overlay.

## Claims

1. A skin dressing comprising a hydrated hydrogel material in combination with a source of oxygen or an oxidising agent.

2. A skin dressing comprising a hydrated hydrogel material and a superposed material that contains a supply of pre-formed hydrogen peroxide or containing a hydrogen peroxide precursor substance.

3. A skin dressing according to claim 1 or claim 2 wherein the hydrated hydrogel material comprises a source of lactate ions and a supply of glucose.

4. A skin dressing according to any one of the preceding claims, further comprising a source of iodide ions.

5. A skin dressing according to any one of the preceding claims, further comprising a source of zinc ions.

6. A skin dressing according to any one of the preceding claims, wherein the hydrated hydrogel material is in the form of a layer, sheet or film of material.

7. A skin dressing according to any one of the preceding claims, wherein the hydrated hydrogel material is in amorphous form.

8. A skin dressing according to any one of the preceding claims, wherein the hydrated hydrogel comprises hydrophilic polymer material.
